# EUROPEAN PATENT APPLICATION

(11) **EP 4 501 964 A1**
(43) Date of publication of application: **05.02.2025**
(21) Application number: 23775361.1
(22) Date of filing: 24.03.2023
(51) Int. Cl.: C07K 16/28, A61P 35/00, A61K 39/00

(54) **HLA-G-SPECIFIC ANTIBODY AND USE THEREOF**

(30) Priority: 25.03.2022 KR 20220037683
(71) Applicant: HK inno.N Corporation, Cheongju-si, Chungcheongbuk-do 28158 (KR); Y-Biologics Inc., Daejeon 34013 (KR)
(72) Inventor: BYUN, Sung Hyun, Seoul 04551 (KR); KIM, Chak Hee, Seoul 04551 (KR); YOON, Da Seul, Seoul 04551 (KR); LEE, Jong Hyun, Seoul 04551 (KR); JUNG, Seung Hee, Seoul 04551 (KR); KANG, Hyeon Ju, Suwon-si Gyeonggi-do 16229 (KR); LEE, Chung Min, Suwon-si Gyeonggi-do 16229 (KR); HA, Gyong Sik, Suwon-si Gyeonggi-do 16229 (KR); YOON, Sun Ha, Daejeon 34014 (KR); KIM, Soo Young, Daejeon 34014 (KR); PARK, Bum-Chan, Daejeon 34014 (KR)
(74) Representative: Maiwald GmbH
(86) International application number: PCT/KR2023/003977
(87) International publication number: WO 2023/182866

(57) **Abstract**

The present invention relates to an antibody specifically binding to HLA-G, and use thereof for cancer treatment, the antibody binding both to an HLA-G monomer and a polymer so as to prevent the binding of HLA-G to a receptor thereof, thereby being effectively used for cancer treatment.

## Description

### [Technical Field]

The present invention relates to an antibody that specifically binds to human leukocyte antigen-G (HLA-G) and a use thereof in cancer treatment.

### [Background Art]

Although intensive research has been conducted on cancer over the past several decades, cancer is still a major cause of death worldwide. Many cancer treatment methods have been developed, and methods such as surgery, chemotherapy, and radiotherapy are still used to this day. However, most of these existing cancer treatment methods are effective only in the early stages when cancer has not metastasized, and have the problem that there is a high possibility of recurrence even after surgery when metastasis has already occurred. Therefore, research on immunotherapy using immune responses has been continuously conducted for more effective cancer treatment.

Immunotherapy is a treatment that prevents cancer cells from evading the human body's immune system, thereby allowing immune cells to better recognize and attack cancer cells. Specifically, immunotherapy may induce the death of cancer cells through non-specific immune cell activation, or induce an immune response against tumors in a cancer patient's immune cells using a tumor-specific antigen.

Meanwhile, human major histocompatibility complex classes I, G, also known as human leukocyte antigen-G (HLA-G), is an immune checkpoint that inhibits the activation and division of T cells, natural killer (NK) cells, and cytotoxic T cells. Known immune cell receptors for HLA-G include Ig-like transcript 2 (ILT2), ILT4 and killer cell immunoglobulin like receptor two Ig domains and long cytoplasmic tail 4 (KIR2DL4), and all of these receptors have an immunoreceptor tyrosine-based inhibitory (ITIM) motif. When HLA-G binds to the receptor, a signaling pathway that suppresses immune cells is activated by the motif.

Although the expression of HLA-G is limited in normal cells, it has been reported that HLA-G is expressed in many types of cancer cells (Lin A, Yan WH. Human Leukocyte Antigen-G (HLA-G) Expression in Cancers: Roles in Immune Evasion, Metastasis and Target for Therapy. Mol Med. 21(1), 782-791 (2015)). In addition, it has been reported that the expression level of HLA-G in cancer cells of many cancer patients is correlated with the stage of cancer, and there is also a report that patients expressing HLA-G have a lower survival rate (Ye, Sr., Yang, H., Li, K. et al. Human leukocyte antigen G expression: as a significant prognostic indicator for patients with colorectal cancer. Mod Pathol 20, 375-383 (2007)).

### [Disclosure]

### [Technical Problem]

In the above situation, the present inventors have tried to develop a human leukocyte antigen-G (HLA-G)-specific antibody to increase the targeting ability of immune cells to cancer cells by suppressing the function of HLA-G. As a result, the present inventors developed an HLA-G-specific antibody capable of binding to both HLA-G monomers and dimers, thereby completing the present invention.

Therefore, an object of the present invention is to provide an HLA-G-specific antibody or an antigen-binding fragment thereof.

In addition, another object of the present invention is to provide a pharmaceutical composition for preventing or treating cancer that provides an HLA-G-specific antibody or an antigen-binding fragment thereof as an active ingredient.

### [Technical Solution]

To achieve the above objects, one aspect of the present invention provides an anti-HLA-G antibody or an antigen-binding fragment thereof including:
(a) a heavy chain complementarity determining region 1 (CDR1) including an amino acid sequence of SEQ ID NO: 1, a heavy chain CDR2 including an amino acid sequence of SEQ ID NO: 2, and a heavy chain CDR3 including an amino acid sequence of SEQ ID NO: 3; and
   a light chain CDR1 including an amino acid sequence of SEQ ID NO: 5, a light chain CDR2 including an amino acid sequence of SEQ ID NO: 6, and a light chain CDR3 including an amino acid sequence of SEQ ID NO: 7;
(b) a heavy chain CDR1 including an amino acid sequence of SEQ ID NO: 9, a heavy chain CDR2 including an amino acid sequence of SEQ ID NO: 10, and a heavy chain CDR3 including an amino acid sequence of SEQ ID NO: 11; and
   a light chain CDR1 including an amino acid sequence of SEQ ID NO: 13, a light chain CDR2 including an amino acid sequence of SEQ ID NO: 14, and a light chain CDR3 including an amino acid sequence of SEQ ID NO: 15;
(c) a heavy chain CDR1 including an amino acid sequence of SEQ ID NO: 17, a heavy chain CDR2 including an amino acid sequence of SEQ ID NO: 18, and a heavy chain CDR3 including an amino acid sequence of SEQ ID NO: 19; and
   a light chain CDR1 including an amino acid sequence of SEQ ID NO: 21, a light chain CDR2 including an amino acid sequence of SEQ ID NO: 22, and a light chain CDR3 including an amino acid sequence of SEQ ID NO: 23; or
(d) a heavy chain CDR1 including an amino acid sequence of SEQ ID NO: 25, a heavy chain CDR2 including an amino acid sequence of SEQ ID NO: 26, and a heavy chain CDR3 including an amino acid sequence of SEQ ID NO: 27; and
   a light chain CDR1 including an amino acid sequence of SEQ ID NO: 29, a light chain CDR2 including an amino acid sequence of SEQ ID NO: 30, and a light chain CDR3 including an amino acid sequence of SEQ ID NO: 31.

In the present invention, the anti-HLA-G antibody or antigen-binding fragment thereof may include the following sequence:
(a) a heavy chain variable region including an amino acid sequence of SEQ ID NO: 4 or an amino acid sequence having 80% or more homology to the amino acid sequence of SEQ ID NO: 4 and a light chain variable region including an amino acid sequence of SEQ ID NO: 8 or an amino acid sequence having 80% or more homology to the amino acid sequence of SEQ ID NO: 8;
(b) a heavy chain variable region including an amino acid sequence of SEQ ID NO: 12 or an amino acid sequence having 80% or more homology to the amino acid sequence of SEQ ID NO: 12 and a light chain variable region including an amino acid sequence of SEQ ID NO: 16 or an amino acid sequence having 80% or more homology to the amino acid sequence of SEQ ID NO: 16;
(c) a heavy chain variable region including an amino acid sequence of SEQ ID NO: 20 or an amino acid sequence having 80% or more homology to the amino acid sequence of SEQ ID NO: 20 and a light chain variable region including an amino acid sequence of SEQ ID NO: 24 or an amino acid sequence having 80% or more homology to the amino acid sequence of SEQ ID NO: 24; or
(d) a heavy chain variable region including an amino acid sequence of SEQ ID NO: 28 or an amino acid sequence having 80% or more homology to the amino acid sequence of SEQ ID NO: 28 and a light chain variable region including an amino acid sequence of SEQ ID NO: 32 or an amino acid sequence having 80% or more homology to the amino acid sequence of SEQ ID NO: 32.

More specifically, in one embodiment of the present invention, the anti-HLA-G antibody or the antigen-binding fragment thereof may include the following sequence:
(a) a heavy chain variable region including an amino acid sequence of SEQ ID NO: 4 and a light chain variable region including an amino acid sequence of SEQ ID NO: 8;
(b) a heavy chain variable region including an amino acid sequence of SEQ ID NO: 12 and a light chain variable region including an amino acid sequence of SEQ ID NO: 16;
(c) a heavy chain variable region including an amino acid sequence of SEQ ID NO: 20 and a light chain variable region including an amino acid sequence of SEQ ID NO: 24; or
(d) a heavy chain variable region including an amino acid sequence of SEQ ID NO: 28 and a light chain variable region including an amino acid sequence of SEQ ID NO: 32.

As used herein, the term "antibody" refers to a protein molecule that serves as a receptor that specifically recognizes an antigen, including an immunoglobulin molecule that immunologically has reactivity to a specific antigen. The antibody includes a monoclonal antibody, a polyclonal antibody, a mixture of monoclonal and/or polyclonal antibodies, a full-length antibody, and an antibody fragment. A full-length antibody or an intact antibody is used interchangeably to refer to an antibody having a structure substantially similar to a native antibody structure, or having a heavy chain containing a fragment crystallizable (Fc) region as defined herein.

In addition, an antibody may include a bivalent or bispecific molecule (e.g., bispecific antibody). In addition, an antibody may be a human antibody, a humanized antibody, or a chimeric antibody according to the origin of the sequence.

As used herein, the term "monoclonal antibody" refers to an antibody molecule of a single molecular composition obtained from a substantially identical antibody population, and such monoclonal antibodies exhibit single binding and affinity for a specific epitope, unlike polyclonal antibodies which may bind to multiple epitopes. As used herein, the term "full-length antibody" has a structure with two full-length light chains and two full-length heavy chains, each light chain being linked to a heavy chain by a disulfide bond. A heavy chain constant region has gamma (γ), mu (µ), alpha (α), delta (δ), and epsilon (ε) types and has gamma 1 (γ1), gamma 2 (γ2), gamma 3 (γ3), gamma 4 (γ4), alpha 1 (α1), and alpha 2 (α2) as subclasses. A light chain constant region has kappa (κ) and lambda (λ) types. IgG has subtypes, including IgG1, IgG2, IgG3, and IgG4.

As used herein, the term "heavy chain" may include both a full-length heavy chain, which includes a variable region VH including an amino acid sequence having a sufficient variable region sequence to impart specificity to an antigen, and three constant regions CH1, CH2, and CH3, and a fragment thereof. In addition, as used herein, the term "light chain" may include both a full-length light chain, which includes a variable region VL including an amino acid sequence having a sufficient variable region sequence to impart specificity to an antigen, and a constant region CL, and a fragment thereof.

As used herein, the term "chimeric antibody" refers to an antibody in which a part of the heavy chain and/or light chain is derived from specific sources or species, whereas the remaining part of the heavy chain and/or light chain is derived from different sources or species. For example, there is a chimeric antibody formed by recombining a variable region of a mouse antibody and a constant region of a human antibody, and it is an antibody with a greatly improved immune response compared to a mouse antibody.

As used herein, the term "humanized antibody" refers to an antibody formed by modifying a protein sequence of an antibody derived from a non-human species to be similar to an antibody naturally produced in a human. For example, a humanized antibody may be produced by recombining a mouse-derived CDR with a human antibody-derived framework region (FR) to produce a humanized variable region, and recombining this with a preferable human antibody constant region. However, when only CDR grafting is performed, the affinity of a humanized antibody decreases, and therefore, by substituting several important FR amino acid residues that are thought to affect the three-dimensional structure of the CDR with those of a mouse antibody, the affinity may be raised to the same level as that of the original mouse antibody.

In addition, the present invention provides an antigen-binding fragment of the anti-HLA-G antibody. The antigen-binding fragment may be selected from the group consisting of fragment antigen binding (Fab), Fab', F(ab')2, a variable fragment (Fv), a disulfide-stabilized Fv fragment (dsFv), a single chain Fv (scFv), a diabody, a triabody and a tetrabody.

As used herein, the terms "fragment," "antibody fragment," "antigen-binding fragment" or "antigen-binding domain" are used interchangeably to refer to any fragment of an antibody of the present invention that has an antigen-binding function of the antibody. Exemplary antigen-binding fragments include Fab, Fab', F(ab')2, and Fv, but are not limited thereto.

The Fab has one antigen binding site with a structure having variable regions of light and heavy chains, a constant region of the light chain, and a first constant region (CH1 domain) of the heavy chain. Fab' is different from Fab in that it has a hinge region including one or more cysteine residues at the C-terminus of the heavy chain CH1 domain. A F(ab')2 antibody is produced when the cysteine residues in the hinge region of Fab' form a disulfide bond. Fv is a minimum antibody fragment having only a heavy chain variable region and a light chain variable region, and recombinant techniques for producing Fv fragments are disclosed in PCT International Patent Publications WO 88/10649, WO 88/106630, WO 88/07085, WO 88/07086, and WO 88/09344, and the like. A two-chain Fv has a heavy chain variable region and a light chain variable region linked by a noncovalent bond, and a single-chain Fv generally has a heavy chain variable region and a light chain variable region linked by a covalent bond via a peptide linker or directly linked at the C-terminus, so that it may form a dimer structure like a two-chain Fv. These antibody fragments may be obtained using a protease (for example, a Fab may be obtained by restriction digestion of a whole antibody with papain, and a F(ab')2 fragment may be obtained by digestion with pepsin), and may preferably be produced by genetic recombination technology.

The diabody is a small bivalent antibody consisting of two heavy chain variable domains and two light chain variable domains, each light chain variable domain being linked to a heavy chain variable domain by a short linker. When the light chain variable domains are linked to the heavy chain variable domains by a much shorter linker, a triabody or tetrabody may be formed.

In addition, the antibody of the present invention or the antigen-binding fragment thereof may include not only the sequence of the anti-HLA-G antibody described herein, but also a biological equivalent thereof, within the range of being able to exhibit binding ability to HLA-G. For example, further changes may be made to the amino acid sequence of the antibody to further improve the binding affinity and/or other biological properties of the antibody. Such modifications include, for example, deletion, insertion and/or substitution of amino acid sequence residues of the antibody. Such amino acid mutations are made based on the relative similarity of the amino acid side chain substituents, such as hydrophobicity, hydrophilicity, charge, size, and the like. By analysis of the size, shape, and type of the amino acid side chain substituents, it can be seen that arginine, lysine, and histidine are all positively charged residues; alanine, glycine, and serine have similar sizes; and phenylalanine, tryptophan, and tyrosine have similar shapes. Therefore, based on this, arginine, lysine, and histidine; alanine, glycine, and serine; and phenylalanine, tryptophan, and tyrosine may be considered as biologically functional equivalents.

Another aspect of the present invention is an isolated nucleic acid encoding the antibody or the antigen-binding fragment thereof. The antibody and the antigen-binding fragment thereof are as described above.

As used herein, the term "nucleic acid" has a comprehensive meaning including DNA (genomic DNA (gDNA) and complementary DNA (cDNA)) and RNA molecules, and nucleotides, which are the basic units of nucleic acid molecules, include not only natural nucleotides but also analogs in which the sugar or base moieties are modified (Scheit, Nucleotide Analogs, John Wiley, New York (1980); Uhlman and Peyman, Chemical Reviews (1990) 90:543-584). The sequence of the nucleic acid molecule encoding the heavy and light chain variable regions of the present invention may be modified, and the modifications include addition, deletion, or non-conservative or conservative substitution of nucleotides.

In the present invention, the isolated nucleic acid may include the following sequence:
(a) a nucleotide sequence of SEQ ID NO: 37 or a nucleotide sequence having 80% or more homology to the nucleotide sequence of SEQ ID NO: 37 and a nucleotide sequence of SEQ ID NO: 38 or a nucleotide sequence having 80% or more homology to the nucleotide sequence of SEQ ID NO: 38;
(b) a nucleotide sequence of SEQ ID NO: 39 or a nucleotide sequence having 80% or more homology to the nucleotide sequence of SEQ ID NO: 39 and a nucleotide sequence of SEQ ID NO: 40 or a nucleotide sequence having 80% or more homology to the nucleotide sequence of SEQ ID NO: 40;
(c) a nucleotide sequence of SEQ ID NO: 41 or a nucleotide sequence having 80% or more homology to the nucleotide sequence of SEQ ID NO: 41 and a nucleotide sequence of SEQ ID NO: 42 or a nucleotide sequence having 80% or more homology to the nucleotide sequence of SEQ ID NO: 42; or
(d) a nucleotide sequence of SEQ ID NO: 43 or a nucleotide sequence having 80% or more homology to the nucleotide sequence of SEQ ID NO: 43 and a nucleotide sequence of SEQ ID NO: 44 or a nucleotide sequence having 80% or more homology to the nucleotide sequence of SEQ ID NO: 44.

More specifically, in one embodiment of the present invention, the isolated nucleic acid may include the following sequence:
(a) a nucleotide sequence of SEQ ID NO: 37 and a nucleotide sequence of SEQ ID NO: 38;
(b) a nucleotide sequence of SEQ ID NO: 39 and a nucleotide sequence of SEQ ID NO: 40;
(c) a nucleotide sequence of SEQ ID NO: 41 and a nucleotide sequence of SEQ ID NO: 42; or
(d) a nucleotide sequence of SEQ ID NO: 43 and a nucleotide sequence of SEQ ID NO: 44.

Still another aspect of the present invention is a recombinant vector including the nucleic acid molecule.

As used herein, the term "vector" refers to a means for expressing a target gene in a host cell, including a plasmid vector; a cosmid vector; and a viral vector such as a bacteriophage vector, an adenovirus vector, a retrovirus vector, and an adeno-associated virus vector, and may preferably be a plasmid vector, but is not limited thereto.

According to one embodiment of the present invention, a recombinant vector including a nucleic acid molecule of the present invention may include a nucleic acid molecule encoding a sequence including the following CDRs or including a heavy chain and/or light chain variable region including the following CDRs:
(a) a heavy chain CDR1 including an amino acid sequence of SEQ ID NO: 1, a heavy chain CDR2 including an amino acid sequence of SEQ ID NO: 2, and a heavy chain CDR3 including an amino acid sequence of SEQ ID NO: 3; and
   a light chain CDR1 including an amino acid sequence of SEQ ID NO: 5, a light chain CDR2 including an amino acid sequence of SEQ ID NO: 6, and a light chain CDR3 including an amino acid sequence of SEQ ID NO: 7;
(b) a heavy chain CDR1 including an amino acid sequence of SEQ ID NO: 9, a heavy chain CDR2 including an amino acid sequence of SEQ ID NO: 10, and a heavy chain CDR3 including an amino acid sequence of SEQ ID NO: 11; and
   a light chain CDR1 including an amino acid sequence of SEQ ID NO: 13, a light chain CDR2 including an amino acid sequence of SEQ ID NO: 14, and a light chain CDR3 including an amino acid sequence of SEQ ID NO: 15;
(c) a heavy chain CDR1 including an amino acid sequence of SEQ ID NO: 17, a heavy chain CDR2 including an amino acid sequence of SEQ ID NO: 18, and a heavy chain CDR3 including an amino acid sequence of SEQ ID NO: 19; and
   a light chain CDR1 including an amino acid sequence of SEQ ID NO: 21, a light chain CDR2 including an amino acid sequence of SEQ ID NO: 22, and a light chain CDR3 including an amino acid sequence of SEQ ID NO: 23; or
(d) a heavy chain CDR1 including an amino acid sequence of SEQ ID NO: 25, a heavy chain CDR2 including an amino acid sequence of SEQ ID NO: 26, and a heavy chain CDR3 including an amino acid sequence of SEQ ID NO: 27; and
   a light chain CDR1 including an amino acid sequence of SEQ ID NO: 29, a light chain CDR2 including an amino acid sequence of SEQ ID NO: 30, and a light chain CDR3 including an amino acid sequence of SEQ ID NO: 31.

In the vector of the present invention, a nucleic acid molecule encoding a light chain variable region and a nucleic acid molecule encoding a heavy chain variable region may be operably linked to a promoter.

As used herein, the term "operably linked" refers to a functional linkage between a nucleic acid expression regulatory sequence (e.g., a promoter, a signal sequence, or an array of transcription factor binding sites) and another nucleic acid sequence, and by this, the regulatory sequence regulates the transcription and/or translation of the other nucleic acid sequence.

The recombinant vector system of the present invention may be constructed by various methods known in the art, and specific methods thereof are disclosed in Sambrook et al., Molecular Cloning, A Laboratory Manual, Cold Spring Harbor Laboratory Press (2001), which is incorporated herein by reference.

The vector of the present invention may typically be constructed as a vector for cloning or as a vector for expression. The recombinant vector of the present invention is preferably an expression vector. In addition, the vector of the present invention may be constructed using a prokaryotic cell or a eukaryotic cell as a host.

For example, when the vector of the present invention is an expression vector and a prokaryotic cell is used as a host, the vector generally includes a strong promoter capable of advancing transcription (e.g., tac promoter, lac promoter, lacUV5 promoter, 1pp promoter, pLλ promoter, pRλ promoter, rac5 promoter, amp promoter, recA promoter, SP6 promoter, trp promoter, T7 promoter, etc.), a ribosome binding site for initiating translation, and a transcription/translation termination sequence. When *E. coli* (e.g., HB101, BL21, DH5α, etc.) is used as a host cell, the promoter and operator regions of the *E. coli* tryptophan biosynthetic pathway (Yanofsky, C., J. Bacteriol., (1984) 158:1018-1024) and the leftward promoter of phage λ (pLλ promoter, Herskowitz, I. and Hagen, D., Ann. Rev. Genet., (1980) 14:399-445) may be used as regulatory regions. When a *Bacillus* bacterium is used as a host cell, the promoter of the toxin protein gene of *Bacillus thuringiensis* (Appl. Environ. Microbiol. (1998) 64:3932-3938; Mol. Gen. Genet. (1996) 250:734-741) or any promoter that may be expressed in a *Bacillus* bacterium may be used as a regulatory region.

Meanwhile, the recombinant vector of the present invention may be produced by manipulating plasmids (e.g., pCL, pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19, etc.), phages (e.g., λgt4·λB, λ-Charon, λΔz1, and M13, etc.) or viruses (e.g., SV40, etc.) often used in the art.

Meanwhile, when the vector of the present invention is an expression vector and a eukaryotic cell is used as a host, a promoter derived from the genome of a mammalian cell (e.g., metallothionine promoter, β-actin promoter, human hemoglobin promoter, and human muscle creatine promoter) or a promoter derived from a mammalian virus (e.g., adenovirus late promoter, vaccinia virus 7.5K promoter, SV40 promoter, cytomegalovirus (CMV) promoter, HSV tk promoter, mouse mammary tumor virus (MMTV) promoter, human immunodeficiency virus long terminal repeat (HIV LTR) promoter, Moloney virus promoter, Epstein-Barr virus (EBV) promoter, and Rous sarcoma virus (RSV) promoter) may be used, and it generally has a polyadenylation sequence as a transcription termination sequence. Specifically, the recombinant vector of the present invention may include a CMV promoter.

The recombinant vector of the present invention may be fused with another sequence to facilitate purification of an antibody expressed therefrom. The fused sequences include, for example, glutathione S-transferase (Pharmacia, USA), maltose binding protein (NEB, USA), FLAG (IBI, USA), and hexahistidine (6x His; Quiagen, USA). In addition, since the protein expressed by the vector of the present invention is an antibody, the expressed antibody may be easily purified through a protein A column, or the like, without an additional sequence for purification.

Meanwhile, the recombinant vector of the present invention may include an antibiotic resistance gene commonly used in the art as a selection marker, and may include, for example, a resistance gene for ampicillin, gentamicin, carbenicillin, chloramphenicol, streptomycin, kanamycin, geneticin, neomycin, and tetracycline. According to one embodiment of the present invention, the anti-HLA-G expression vector may include an ampicillin resistance gene.

The vector expressing the antibody of the present invention may be a vector system in which the light chain and the heavy chain are simultaneously expressed in a single vector, or a system in which the light chain and the heavy chain are each expressed in separate vectors. In the latter case, the two vectors are introduced into a host cell through co-transformation and targeted transformation. Co-transformation is a method in which respective vector DNAs encoding the light chain and the heavy chain are simultaneously introduced into a host cell, and then cells expressing both the light chain and the heavy chain are selected. Targeted transformation is a method in which cells transformed with a vector including a light chain (or heavy chain) are selected, and the selected cells expressing the light chain are transformed again with a vector including a heavy chain (or light chain) to finally select cells expressing both the light chain and the heavy chain.

The nucleic acid encoding the heavy chain and light chain of the antibody of the present invention is inserted into an expression vector encoding the heavy chain constant region and the light chain constant region of human IgG1, IgG2, IgG4. The light chain and the heavy chain may be cloned in the same or different expression vectors. The DNA sequence encoding the immunoglobulin chain is operably linked to a control sequence in the expression vector(s) that ensures the expression of the immunoglobulin polypeptide. Such control sequences include a signal sequence, a promoter, an enhancer, and a transcription termination sequence. The expression vector is an integral part of the host chromosomal DNA or an episome and is replicable in the host organism.

When the heavy and light chains of an antibody are respectively cloned into separate vectors, expression vectors encoding the heavy chain and the light chain may be co-transfected into a single host cell for the expression of both chains, and the chains may be assembled to form an intact antibody *in vivo* or *in vitro.*

Alternatively, an expression vector encoding the heavy chain and an expression vector encoding the light chain may be introduced into different host cells for respective expression of the heavy and light chains, and the chains may then be purified and assembled *in vitro* to form intact antibodies. The antibody or the fragment thereof as described herein may be produced in a prokaryotic or eukaryotic expression system, such as bacteria, yeast, filamentous fungi, insects, and mammalian cells. Although the recombinant antibodies of the present invention need not to necessarily be glycosylated or expressed in a eukaryotic cell, expression in mammalian cells is generally preferable. Examples of useful mammalian host cell lines are human embryonic kidney cell lines (HEK 293 cells), baby hamster kidney cells (BHK cells), Chinese hamster ovary cells/- or + dihydrofolate reductase (DHFR) (CHO, CHO-S, CHO-DG44, Flp-in CHO cells), African green monkey kidney cells (VERO cells), and human hepatic cells (Hep G2 cells).

Expression vectors for these cells may include expression control sequences such as a replication origin, a promoter, and an enhancer, and necessary processing information sites such as a ribosome binding site, an RNA splice site, a polyadenylation site, and a transcription terminator sequence. Preferred expression control sequences are promoters derived from immunoglobulin genes, simian virus 40 (SV40), adenovirus, bovine papilloma virus, cytomegalovirus, and the like. A vector containing a plasmid sequence for producing an antibody may be transferred into a host cell by well-known methods, which may vary depending on the type of host cell.

Yet another aspect of the present invention is a host cell including the recombinant vector. Preferably, the host cell of the present invention is a host cell transformed with the recombinant vector.

The host cell capable of stably and continuously cloning and expressing the vector of the present invention may be any host cell known in the art and may include, for example, prokaryotic host cells such as *Escherichia coli,* strains of the genus *Bacillus* such as *Bacillus subtilis* and *Bacillus thuringiensis, Streptomyces, Pseudomonas* (e.g., *Pseudomonas putida), Proteus mirabilis,* or *Staphylococcus* (e.g., *Staphylococcus carnosus),* but is not limited thereto.

Suitable eukaryotic host cells transformed with the vector may be fungi such as the genus *Aspergillus* species, yeasts such as *Pichia pastoris, Saccharomyces cerevisiae, Schizosaccharomyces,* and *Neurospora crassa,* other lower eukaryotic cells, higher eukaryotic cells such as insect-derived cells, and cells derived from plants or mammals, but are not limited thereto.

Specifically, the host cell may be monkey kidney cells 7 (COS7), NS0 cells, SP2/0, CHO cells, W138, BHK cells, Mardin Darby canine kidney (MDCK) cells, myeloma cell lines, HuT 78 cells, or 293 cells.

Mammalian host cell cultures are preferable for expressing and producing an anti-HLA-G antibody or an antigen-binding fragment thereof, because a number of suitable host cell lines capable of secreting intact immunoglobulins have been developed in the art.

In the present invention, "transformation" and/or "transfection" into a host cell includes any method for introducing a nucleic acid into an organism, cell, tissue, or organ, and may be performed by selecting a standard technique suitable for the host cell as known in the art. Such methods include electroporation, protoplast fusion, calcium phosphate (CaPO₄) precipitation, calcium chloride (CaCl₂) precipitation, stirring using silicon carbide fibers, *Agrobacterium*-mediated transformation, polyethylene glycol (PEG), dextran sulfate, lipofectamine, and drying/inhibition-mediated transformation methods, but are not limited thereto.

Yet another aspect of the present invention is a method for producing an anti-HLA-G antibody or an antigen-binding fragment thereof, including culturing a transformed host cell of the present invention. Preferably, the method for producing the anti-HLA-G antibody or the antigen-binding fragment thereof of the present invention may further include expressing the anti-HLA-G antibody or the antigen-binding fragment thereof in a cultured transformed host cell.

In the method for producing the antibody or the antigen-binding fragment thereof, the culturing of a transformed host cell may be performed according to appropriate media and culture conditions known in the art. Such culture process may be easily adjusted and used by those skilled in the art according to the selected strain. These various culture methods are disclosed in various documents (e.g., James M. Lee, Biochemical Engineering, Prentice-Hall International Editions, 138-176). Cell culture is classified into suspension culture and adherent culture according to the cell growth method, and into batch, fed-batch, and continuous culture methods according to the culture method. The medium used for culture needs to appropriately satisfy the requirements of a specific strain.

In animal cell culture, the medium contains various carbon sources, nitrogen sources, and trace element ingredients. Examples of carbon sources that may be used include carbohydrates such as glucose, sucrose, lactose, fructose, maltose, starch, and cellulose, fats such as soybean oil, sunflower oil, castor oil, and coconut oil, fatty acids such as palmitic acid, stearic acid, and linoleic acid, alcohols such as glycerol and ethanol, and organic acids such as acetic acid, and these carbon sources may be used alone or in combination.

Nitrogen sources that may be used in the present invention include organic nitrogen sources such as peptone, yeast extract, meat juice, malt extract, corn steep liquor (CSL), and soybean meal, and inorganic nitrogen sources such as urea, ammonium sulfate, ammonium chloride, ammonium phosphate, ammonium carbonate, and ammonium nitrate, and these nitrogen sources may be used alone or in combination. The medium may contain, as a phosphorus source, potassium dihydrogen phosphate, dipotassium hydrogen phosphate, and corresponding sodium-containing salts. In addition, the medium may contain metal salts such as magnesium sulfate or iron sulfate. In addition, amino acids, vitamins, and appropriate precursors may be contained.

During the culture, compounds such as ammonium hydroxide, potassium hydroxide, ammonia, phosphoric acid, and sulfuric acid may be added to the culture in an appropriate manner to adjust the pH of the culture. In addition, during the culture, an antifoaming agent such as fatty acid polyglycol ester may be used to suppress bubble formation. In addition, to maintain the aerobic conditions of the culture, oxygen or an oxygen-containing gas (e.g., air) is injected into the culture. The temperature of the culture may usually be 20 °C to 45 °C, preferably 25 °C to 40 °C, but is not limited thereto.

The antibody obtained by culturing the transformed host cell may be used in an unpurified state, and may be further purified to high purity using various conventional methods, such as dialysis, salt precipitation, and chromatography. Among them, the method using chromatography is the most commonly used, and the type and order of columns may be selected from ion exchange chromatography, size exclusion chromatography, and affinity chromatography according to the characteristics of the antibody, the culturing method, and the like.

The anti-HLA-G antibody according to the present invention is produced using the multimeric structure of HLA-G as an antigen, and is characterized in that it is able to bind to both HLA-G monomers and multimers. Since HLA-G is known to suppress the activation of immune cells and to be overexpressed in cancer cells, the present inventors confirmed the anticancer ability of the anti-HLA-G antibody.

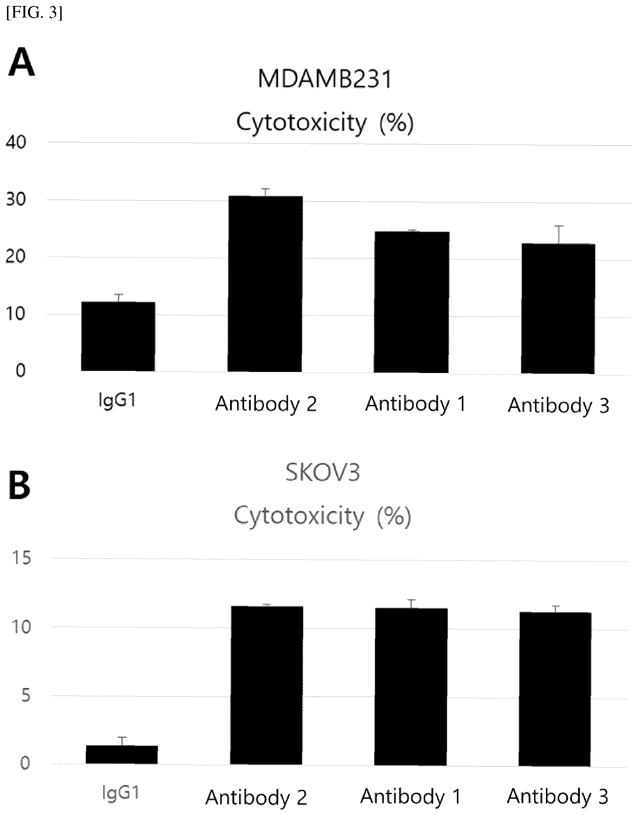

As a result, it was confirmed that the anti-HLA-G antibody inhibited the binding of HLA-G to CD8a (Table 4). In addition, it was confirmed that when peripheral blood mononuclear cells (PBMCs, effector cells) and target cells treated with the anti-HLA-G antibody were mixed and co-cultured with cancer cells, the cancer cells were killed (FIG. 3) and tumor growth was inhibited in tumor-induced mice (Table 6).

Accordingly, yet another aspect of the present invention provides a pharmaceutical composition for use in the prevention or treatment of cancer, including the antibody of the present invention or the antigen-binding fragment thereof as an active ingredient.

In the present invention, the pharmaceutical composition may be used for the treatment of cancer that overexpresses HLA-G, but is not limited thereto. The cancer may be selected from the group consisting of pancreatic cancer, breast cancer, ovarian cancer, glioma, cervical cancer, endometrial cancer, esophageal cancer, stomach cancer, liver cancer, lung cancer, colon cancer, nasopharyngeal cancer, oral cancer, thyroid cancer, prostate cancer, renal cancer, gallbladder cancer, bile duct cancer, blood cancer, melanoma, and the like.

The blood cancer may be acute myeloid leukemia (AML), acute lymphoblastic leukemia (ALL), chronic myelogenous leukemia (CML), multiple myeloma (MM), or lymphoma.

As used herein, the term "prevention" means any act of suppressing or delaying the progression of a disease by administering the composition of the present invention, and "treatment" means suppressing, alleviating, or eliminating the development of a disease.

The pharmaceutical composition of the present invention may further include a pharmaceutically acceptable carrier, and the pharmaceutically acceptable carrier is one that is commonly used in the preparation of formulations, and includes lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methyl hydroxybenzoate, propyl hydroxybenzoate, talc, magnesium stearate, and mineral oil, but is not limited thereto. In addition to the above ingredients, the composition for preventing or treating cancer of the present invention may further include a lubricant, a wetting agent, a sweetening agent, a flavoring agent, an emulsifier, a suspending agent, a preservative, and the like. Suitable pharmaceutically acceptable carriers and formulations are described in detail in the document [Remington's Pharmaceutical Sciences (19th ed., 1995)].

The pharmaceutical composition of the present invention may be administered orally or parenterally, and in the case of parenteral administration, it may be administered by intravenous injection, subcutaneous injection, intramuscular injection, intraperitoneal injection, intradermal administration, topical administration, intranasal administration, intrapulmonary administration, and intrarectal administration, and the like. In the case of oral administration, since proteins or peptides are digested, the oral composition may be formulated to coat the active agent or protect it from decomposition in the stomach, and the composition of the present invention may be administered by any device that allows the active substance to move to the target cell.

The suitable dosage of the pharmaceutical composition of the present invention varies depending on factors such as the formulation method, administration method, the patient's age, weight, sex, and pathological conditions, food, administration time, administration route, excretion rate, and response sensitivity, and a physician of ordinary skill may easily determine and prescribe a dosage effective for the desired treatment or prevention. According to one embodiment of the present invention, the daily dosage of the pharmaceutical composition of the present invention may be 0.1 to 100 mg/kg, preferably 0.1 to 10 mg/kg, and more preferably 0.1 to 2 mg/kg. As used herein, the term "pharmaceutically effective amount" refers to an amount sufficient for the treatment, prevention, and diagnosis of a disease.

The pharmaceutical composition of the present invention may be prepared in a unit dosage form or it may be prepared by placing it into a multi-dose container by formulating it using a pharmaceutically acceptable carrier and/or excipient according to a method that may be easily implemented by a person skilled in the art to which the present invention pertains. At this time, the formulation may be in the form of a solution, suspension or emulsion in an oil or an aqueous medium, or it may be in the form of an extract, powder, granules, tablets, or capsules, and may further contain a dispersant and/or stabilizer.

The composition of the present invention may be administered a subject therapeutic agent or in combination with other therapeutic agents, and may be administered sequentially or simultaneously with conventional therapeutic agents.

The antibody or the antigen-binding fragment thereof of the present invention may be used for the treatment of cancer by administering it into a living body in the form of antibody-therapeutic (functional molecule) and bispecific antibody-therapeutic (functional molecule) conjugates. Various conditions suitable and preferable for targeting a drug to a specific target site have been reported, for example, in the document [Trouet et al., Plenum Press, New York and London, (1982) 19-30].

The antibody or the antigen-binding fragment thereof of the present invention may be further combined with or co-administered with a functional molecule to be used for the prevention, treatment, and diagnosis of a disease associated with HLA-G overexpression. The functional molecule may include a chemical substance, a radionuclide, an immunotherapeutic, a cytokine, a chemokine, a toxin, a biological agent, and an enzyme inhibitor.

Yet another aspect of the present invention provides a method of diagnosing, preventing, or treating a disease associated with HLA-G overexpression, for example, cancer, the method including administering the antibody or the antigen-binding fragment thereof of the present invention, the pharmaceutical composition in a pharmaceutically effective amount to a subject, for example, a human or a non-human mammal. The present invention also provides a method of diagnosing a disease associated with HLA-G overexpression, for example, cancer, the method including administering the antibody or the antigen-binding fragment thereof of the present invention to a subject, for example, a human or a non-human mammal, in need thereof.

As used herein, the term "subject" refers to any animal, including a human, a monkey, a cow, a horse, a sheep, a pig, a chicken, a turkey, a quail, a cat, a dog, a mouse, a rat, a rabbit, or a guinea pig, which has developed or may develop a disease associated with HLA-G overexpression.

Meanwhile, the present inventors confirmed the binding site of the antibody and HLA-G and, as a result, confirmed that in the amino acid sequence (SEQ ID NO: 33) of the antigen used for antibody screening, amino acids 339 to 344 (DYEATL), amino acids 390 to 402 (VVVPSGEEQRYTC), and amino acids 323 to 338 (QRADPPKTHVTHHPVF) were epitopes.

Therefore, yet another aspect of the present invention provides an HLA-G epitope consisting of a sequence selected from the group consisting of SEQ ID NOs: 34 to 36.

As used herein, the term "epitope" refers to a localized site of an antigen to which an antibody or a fragment thereof may specifically bind. An epitope is usually composed of surface groups of molecules, such as amino acids or sugar side chains, and generally has specific three-dimensional structural characteristics and specific charge characteristics. Conformational and non-conformational epitopes are distinguished in that binding to a conformational epitope is lost in the presence of a denaturing solvent, whereas binding to a non-conformational epitope is not lost. An epitope may include amino acid residues that are directly involved in binding (also referred to as immunodominant component of the epitope) and other amino acid residues that are not directly involved in binding, such as amino acid residues that are effectively blocked by a specific antigen-binding peptide (i.e., the amino acid residues are present within the footprint of the specific antigen-binding peptide).

The present invention also provides an antibody that specifically binds to the HLA-G epitope, and specific examples of the antibody are the same as described above, but are not limited thereto.

### [Advantageous Effects]

The anti-HLA-G antibody according to the present invention can bind to both monomers and multimers of HLA-G, and thus can effectively block the binding of HLA-G to a receptor thereof.

### [Description of Drawings]

FIG. 1 is a schematic diagram for producing a b2m-HLA-G antigen according to an embodiment of the present invention.
FIG. 2 shows a vector structure used for producing an anti-HLA-G antibody.
FIG. 3 shows the results of mixing HLA-G overexpressing target cells treated with an anti-HLA-G antibody with peripheral blood mononuclear cells (PBMCs), co-culturing the mixed cells with SK-OV-3 cells or MDA-MB-231 cells, and confirming the degree of cancer cell death.

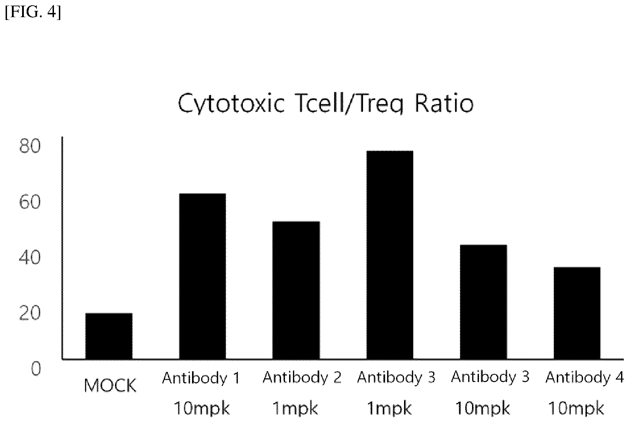

FIG. 4 shows the results of transplanting 4T-1 cells overexpressing human HLA-G into mice to induce tumors, administering the anti-HLA-G antibody, and isolating the tumors to confirm the levels of cytotoxic T cells/regulatory T cells.
FIG. 5 shows the results of confirming the epitope sequence at which the anti-HLA-G antibody binds to HLA-G.

### [Modes of the Invention]

Hereinafter, one or more embodiments are described in more detail through examples. However, these examples are provided for illustrative purposes only, and the scope of the present invention is not limited to these examples.

### Example 1: Antigen design

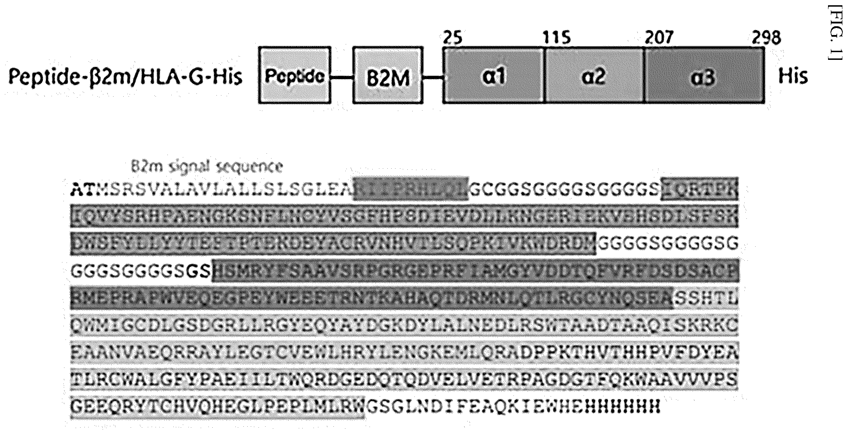

To discover an HLA-G-specific antibody and to inhibit the action mechanism of HLA-G, an antigen similar to the b2m-HLA-G multimer structure, which is an activated form of HLA-G, was produced. To easily produce the antigen, a protein sequence of linked signal peptide-b2m-HLA-G-6X His was designed. A DNA sequence was designed using this protein sequence (FIG. 1). Subsequently, the DNA sequence was introduced into animal cells, and the protein was expressed and purified. Specifically, a cell culture solution containing the signal peptide-b2m-HLA-G-6X His antigen was primarily purified using a nickel-nitrilotriacetic acid (NI-NTA) column. Thereafter, a monomer and a dimer were separated from the resulting product of the primary purification using size exclusion chromatography. Antigen production is not limited to the above-described animal cells, and it is also possible to express and purify in, for example, *E. coli.*

### SEQ ID NO: 33: Antigen sequence

MSRSVALAVLALLSLSGLEARIIPRHLQLGCGGSGGGGSGGGGSIQRTPKI
QVYSRHPAENGKSNFLNCYVSGFHPSDIEVDLLKNGERIEKVEHSDLSFSK
DWSFYLLYYTEFTPTEKDEYACRVNHVTLSQPKIVKWDRDMGGGGSGGG
GSGGGGSGGGGSGSHSMRYFSAAVSRPGRGEPRFIAMGYVDDTQFVRFD
SDSACPRMEPRAPWVEQEGPEYWEEETRNTKAHAQTDRMNLQTLRGCY
NQSEASSHTLQWMIGCDLGSDGRLLRGYEQYAYDGKDYLALNEDLRSW
TAADTAAQISKRKCEAANVAEQRRAYLEGTCVEWLHRYLENGKEMLQR
ADPPKTHVTHHPVFDYEATLRCWALGFYPAEIILTWQRDGEDQTQDVELV
ETRPAGDGTFQKWAAVVVPSGEEQRYTCHVQHEGLPEPLMLRWGSGLN
DIFEAQKIEWHEHHHHHH

### Example 2: Antibody production

### 2-1. Library screening

Screening of the phage/scFv library was performed using an immunotube (15501, Piece) coated with the HLA-G antigen. Panning was performed four times to increase the antigen binding specificity of the phage/scFv library. For the recovery and amplification of the selected phage, exponentially growing *E. coli* cultures were infected with the phage suspension eluted after each round of panning. After the completion of the panning, a pep-phage-enzyme-linked immunosorbent assay (ELISA) was performed using the HLA-G antigen to evaluate the antigen reactivity of the selected phage. After the fourth round of panning, the antigen reaction signal of the phage increased up to 3 times the background. According to phage display technology, the signal increase as described above indicates that the selected phage is rich in HLA-G antigen-specific conjugates. After the fourth round of panning, the phagemid DNA of the selected phage was extracted from the library. Ninety six clones were isolated and cultured on 24-well plates. The supernatant containing the phage particles was tested by phage-ELISA, and the DNA of the clones exhibiting HLA-G-specific binding was isolated and sequenced.

### 2-2. Antibody maturation

For the maturation of the HLA-G-specific antibody, mutations were introduced into CDR1, 2, and 3 of the heavy chain and light chain to obtain various heavy chain and light chain products. The products were combinated, and HLA-G-specific mature antibodies were obtained through an ELISA analysis using HLA-A, HLA-B, HLA-C, and HLA-G to select HLA-G-specific antibodies.

### 2-3. Recombinant production of antibody

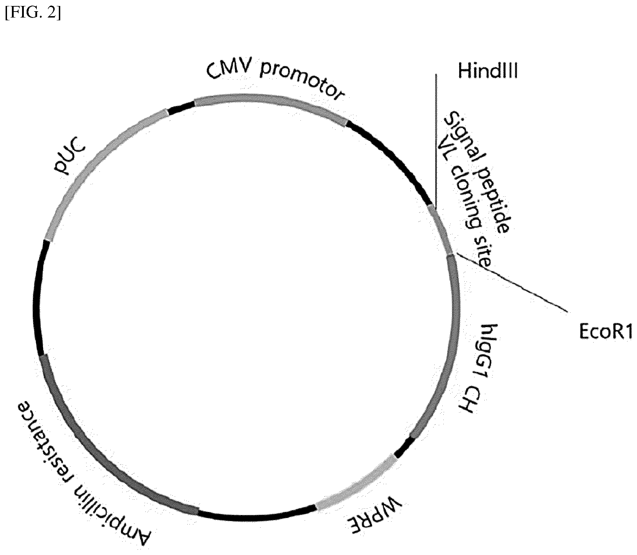

In the present example, antibodies were produced using the vector structure of FIG. 2. The vector includes a human CMV promoter for expression in animal cells, a Kozak sequence for expression enhancement, a signal sequence for extracellular transport of expressed proteins, a variable region cloning site, an IgG1 light chain or heavy chain constant region coding sequence, a Woodchuck hepatitis virus posttranscriptional regulatory element (WPRE), an ampicillin resistance gene, and pUC for amplifying the plasmid in *E. coli.* Since the variable region cloning site had HindIII and EcoR1 restriction enzyme sites, the expression vector was treated with the restriction enzymes, and then the polymerase chain reaction (PCR) product of the heavy chain or light chain variable region was inserted into the vector using an infusion enzyme (TAKARA). The heavy chain constant domains of IgG2, IgG3, and IgG4 may also be used.

The light chain expression vector and the heavy chain expression vector were transduced into Expi 293 cells using a transduction kit (Thermo), and then the cells were cultured. The cell culture was harvested four to six days later, and the cells were removed to collect only the supernatant. The collected supernatant was filtered through a 0.45 µm filter, and the filtered culture solution was purified using a Mabselect (Cytiva) purification column in an ÄKTA purification device to obtain an antibody that specifically binds to human HLA-G.

**2-4. Antibody analysis**

The obtained antibodies were analyzed, and the CDR and variable region sequences are summarized in Tables 1 and 2 below.

**[Table 1]**

| Anti-HLAG antibody (Sequence ID number of CDR) | | | | | | | | |
|---|---|---|---|---|---|---|---|---|
| Anti-HLAG antibody | CDR-H1 | CDR-H2 | CDR-H3 | VH | CDR-L1 | CDR-L2 | CDR-L3 | VL |
| Antibody 1 | SEQ ID NO: 1 | SEQ ID NO: 2 | SEQ ID NO: 3 | SEQ ID NO: 4 | SEQ ID NO: 5 | SEQ ID NO: 6 | SEQ ID NO: 7 | SEQ ID NO: 8 |
| Antibody 2 | SEQ ID NO: 9 | SEQ ID NO: 10 | SEQ ID NO: 11 | SEQ ID NO: 12 | SEQ ID NO: 13 | SEQ ID NO: 14 | SEQ ID NO: 15 | SEQ ID NO: 16 |
| Antibody 3 | SEQ ID NO: 17 | SEQ ID NO: 18 | SEQ ID NO: 19 | SEQ ID NO: 20 | SEQ ID NO: 21 | SEQ ID NO: 22 | SEQ ID NO: 23 | SEQ ID NO: 24 |
| Antibody 4 | SEQ ID NO: 25 | SEQ ID NO: 26 | SEQ ID NO: 27 | SEQ ID NO: 28 | SEQ ID NO: 29 | SEQ ID NO: 30 | SEQ ID NO: 31 | SEQ ID NO: 32 |

**[Table 2]**

| Antibody 1 VH | | |
|---|---|---|
| CDR1 | SEQ ID NO:1 | DYAMS |
| CDR2 | SEQ ID NO:2 | VISHDGDRVYYADSVKG |
| CDR3 | SEQ ID NO:3 | GPRRIANAIFDY |
| VH full | SEQ ID NO:4 | EVQLLESGGGLVQPGGSLRLSCAASGFTFSDYAMSWVRQAPGKGLEWV SVISHDGDRVYYADSVKGRFTISRDNSKNTLYLQMNSLRAEDTAVYYC AKGPRRIANAIFDYWGQGTLVTVSS |

| Antibody 1 VL | | |
|---|---|---|
| CDR1 | SEQ ID NO:5 | SGSSSNIGHSYVS |
| CDR2 | SEQ ID NO:6 | GDNNRPS |
| CDR3 | SEQ ID NO:7 | QSYDSSLSGYV |
| VL full | SEQ ID NO:8 | QLVLTQPPSVSGAPGQKVTISCSGSSSNIGHSYVSWYQQLPGTAPKLLIY GDNNRPSGVPDRFSGSKSGTSASLAITGLQAEDEADYYCQSYDSSLSGY VFGTGTKVTVL |

| Antibody 2 VH | | |
|---|---|---|
| CDR1 | SEQ ID NO:9 | DYAMS |
| CDR2 | SEQ ID NO:10 | VISHDGGSIYYADSVKG |
| CDR3 | SEQ ID NO:11 | GPRRIANAIFDY |
| VH full | SEQ ID NO:12 | EVQLLESGGGLVQPGGSLRLSCAASGFTFSDYAMSWVRQAPGKGLEWV SVISHDGGSIYYADSVKGRFTISRDNSKNTLYLQMNSLRAEDTAVYYCA KGPRRIANAIFDYWGQGTLVTVSS |

| Antibody 2 VL | | |
|---|---|---|
| CDR1 | SEQ ID NO:13 | SGSNSNIGNSYVS |
| CDR2 | SEQ ID NO:14 | GSTNRPS |
| CDR3 | SEQ ID NO:15 | QSYDSSLSGYV |
| VL full | SEQ ID NO:16 | QFVLTQPPSVSAAPGQKVTISCSGSNSNIGNSYVSWYQQVPGAAPRLLIY |
| | | GSTNRPSGVPDRFSGSKSGTSASLAITGLQAEDEADYYCQ SYDSSLSGYVFGTGTKVTVL |

| Antibody 3 VH | | |
|---|---|---|
| CDR1 | SEQ ID NO:17 | DYSMS |
| CDR2 | SEQ ID NO:18 | AISPDRSLEYYADSVKG |
| CDR3 | SEQ ID NO:19 | GPRHLTNNIFDY |
| VH full | SEQ ID NO:20 | EVQLLESGGGLVQPGGSLRLSCAASGFTFSDYSMSWVRQAPGKGLEWV SAISPDRSLEYYADSVKGRFTISRDNSKNTLYLQMNSLRAEDTAVYYCA KGPRHLTNNIFDYWGQGTLVTVSS |

| Antibody 3 VL | | |
|---|---|---|
| CDR1 | SEQ ID NO:21 | SGSSSNIGHSYVS |
| CDR2 | SEQ ID NO:22 | GNIHRPS |
| CDR3 | SEQ ID NO:23 | GVWDSSLSAVV |
| VL full | SEQ ID NO:24 | QFVLTQPPSVSAAPGQKVTISCSGSSSNIGHSYVSWYQQLPGTAPKLLIY GNIHRPSGVPDRFSGSTSGTSASLAITGLQADDEADYYCG VWDSSLSAVVFGGGTKLTVL |

| Antibody 4 VH | | |
|---|---|---|
| CDR1 | SEQ ID NO:25 | DYSMS |
| CDR2 | SEQ ID NO:26 | AISPDSGSKYYADSVKG |
| CDR3 | SEQ ID NO:27 | GPRHLTNNIFDY |
| VH full | SEQ ID NO:28 | EVQLLESGGGLVQPGGSLRLSCAASGFTFSDYSMSWVRQAPGKGLEWV SAISPDSGSKYYADSVKGRFTISRDNSKNTLYLQMNSLRAEDTAVYYCA KGPRHLTNNIFDYWGQGTLVTVSS |

| Antibody 4 VL | | |
|---|---|---|
| CDR1 | SEQ ID NO:29 | TGSSSNIGSNYVS |
| CDR2 | SEQ ID NO:30 | ADSNRPS |
| CDR3 | SEQ ID NO:31 | ASWDYSLSGYV |
| VL full | SEQ ID NO:32 | QSVLTQPPSASGTPGQRVTISCTGSSSNIGSNYVSWYQQLPGTAPKLLIY ADSNRPSGVPDRFSGSKSGTSASLAISGLRSEDEADYYCASWDYSLSGY VFGGGTKLTVL |

### Example 3: Evaluation of antigen binding ability of antibodies

The binding properties of the anti-HLA-G specific antibodies obtained in Example 2 to the HLA-G antigen produced in Example 1 were screened. The analytical results are summarized in Table 3 below.

**[Table 3]**

| Anti-HLA-G antibody | SPR KD (M) | | Octet KD (M) |
|---|---|---|---|
| | HLA-G monomer | HLA-G dimer | |
| Antibody 1 | 1.5 E-08 | 1.11 E-09 | 2.84 E-10 |
| Antibody 2 | 9.30 E-09 | 8.41 E-10 | 5.49 E-10 |
| Antibody 3 | 2.81 E-08 | 3.98 E-09 | 1.06 E-9 |
| Antibody 4 | - | 4.88 E-09 | 1.45 E-9 |

Through surface plasmon resonance (SPR) and octet analysis, it was confirmed that the anti-HLA-G specific antibodies had high affinity for the antigen, as they exhibited nanomolar KD values for HLA-G multimers. In addition, the anti-HLA-G specific antibodies, which were obtained from HLA-G multimers, exhibited higher affinity for the multimers than for the monomers.

### Example 4: Confirmation of binding inhibition ability of HLA-G specific antibodies

Since the anti-HLA-G specific antibodies have high affinity for HLA-G multimers, it was confirmed whether these antibodies could inhibit the binding between Ig-like transcript 2 (ILT2) and HLA-G multimers.

### 4-1. Receptor binding inhibition 1: Inhibition of ILT2 binding to HLA-G by anti-HLA-G antibodies

### a. ILT2 Block ELISA

For analysis, ILT-2-Fc was diluted to a concentration of 1.5 µg/mL in 1x Dulbecco's phosphate buffered saline (DPBS). The diluted ILT-2 protein was dispensed into each well of a 96-well immune plate in an amount of 150 ng/100 µL. The immune plate was stored overnight at 4 °C to immobilize the ILT-2 protein to the surface of the well. The next day, the ILT-2 protein solution was completely removed, and each well was washed with 200 µL of 1x phosphate buffered saline with Tween (PBS-T). After dispensing 200 µL of blocking solution into each well, the reactants were allowed to react at 37 °C for one hour to block nonspecific protein binding.

The antigen produced in Example 1 was diluted with a blocking solution to 1,200 nM, which was twice the concentration to be treated. In addition, the anti-HLA-G single antibody was diluted with a blocking solution to 2,000 nM, which was twice the concentration to be treated, and then serially diluted three-fold to 0.914 nM.

The prepared antigen of Example 1 and the anti-HLA-G single antibody diluted to different concentrations were mixed at a 1:1 ratio, and 120 µl of the resulting mixture was dispensed into each well and pre-incubated at 37 °C for 30 minutes. After completely removing the blocking solution from each well, the wells were washed with PBS-T.

The 1:1 mixture of the antigen of Example 1 and anti-HLA-G single antibody was added to each well in an amount of 100 µl and allowed to react at 37 °C for one hour. Anti-His-horseradish peroxidase (HRP), which is a detection antibody against the antigen of Example 1, was diluted at a ratio of 1:10,000, dispensed into each well in an amount of 100 µl, and the resulting mixture was allowed to react at 37 °C for one hour. Each well was washed three times with 200 µl of 1x PBS-T, and the absorbance was measured at 450 nm using a multimode microplate reader.

The measurement results are shown in Table 4. Table 4 below shows the ELISA analysis results of confirming the concentration of antibodies that inhibited the HLA-G:ILT2 interaction by 50% or more when the unblocked HLA-G:ILT2 interaction was treated with different concentrations of antibodies. The antibody obtained in Example 2 was found to inhibit HLA-G:ILT2 binding by 50% or more at the 150 nM level.

**[Table 4]**

| Anti-HLA-G antibody | ILT2 Block EIA (nM) | ILT-2 Block Cell (nM) | CD8a Block EIA (%) |
|---|---|---|---|
| Antibody 1 | 154.6 | 0.3204 | 39.5 |
| Antibody 2 | 146.4 | 0.2156 | 39 |
| Antibody 3 | 163.7 | 2.743 | 37.7 |
| Antibody 4 | 143.5 | 2.837 | 32.1 |

### b. ILT-2 Block Cell

HLA-G expressing cells were seeded in a 96-well microplate at a density of 3.0. E+05 cells/well on day 0 (D0) of the experiment and cultured. The cell supernatant was removed on day 1 of the experiment. The anti-HLA-G single antibody was diluted with a blocking solution to 200 nM, which is twice the concentration to be treated, and serially diluted 6-fold to 0.714 pM. This diluted solution was dispensed into the well from which the supernatant had been removed and allowed to react for 30 minutes. The ILT2-mFc protein was diluted to 100 µg/mL, and the diluted solution was dispensed into the antibody-cell reaction solution and allowed to react for one hour. After the reaction was completed, the supernatant was removed, and anti-mouse IgG-fluorescein-5-isothiocyanate (FITC), which is capable of detecting ILT2-mFc, was diluted and dispensed. After a reaction for two hours, the residual amount of ILT2-mFc was confirmed using a flow cytometer.

According to the confirmation results, the negative control group, which was the IgG1-treated group, did not inhibit the binding of HLA-G and ILT2 even at high concentrations, but the antibody obtained in Example 2 inhibited the binding of HLA-G and ILT2 in a concentration-dependent manner. In addition, the antibody concentration (EC50) at which the HLA-G expressing cell:ILT2 binding was 50% or less was confirmed, and it was found that the obtained antibody effectively inhibited the binding of HLA-G and ILT2 on the cell surface at nanomolar levels (Table 4).

### 4-2. Receptor binding inhibition 2: Inhibition of CD8a binding to HLA-G by anti-HLA-G antibodies

When the HLA-G antigen binds to CD8a expressed in immune cells, the inhibition of immune cells is exhibited, and thus inhibiting the binding of HLA-G and CD8a is important for the activation of immune cells. Accordingly, the CD8a blocking effect of the antibodies obtained in Example 2 was confirmed as described below.

The dimeric antigen produced in Example 1 was diluted to a concentration of 5 µg/mL in a PBS solution. The diluted antigen was dispensed into a 96-well microplate in an amount of 100 µl for each well to coat the wells with the antigen for 24 hours.

A blocking solution was dispensed into each well of a streptavidin 96-well plate in an amount of 100 µl for each well for blocking. The blocking solution was prepared by dissolving skim milk at a concentration of 4% in PBS containing 1% Tween-20. Each well of the streptavidin 96-well plate was washed three times with 100 µL of PBS containing 0.1% Tween-20.

Thereafter, an anti-HLA-G antibody was diluted with the blocking solution to a final concentration of 0.391 nM. The diluted anti-HLA-G antibody was dispensed into 96 wells and allowed to react for one hour. CD8a-His at a concentration of 100 nM diluted with the blocking solution was dispensed into each well and allowed to react for one hour. After the reaction was completed, each well was washed with PBS-T. Anti-CD8a-HRP, which is a CD8a detection antibody, was diluted with the blocking solution and dispensed into each well. After a reaction for one hour, each well was washed with PBS-T, and a tetramethylbenzidine (TMB) solution was added to each well to develop color. The reaction was terminated by adding 1 N sulfuric acid, and the absorbance was measured at 450 nM using a microplate reader.

According to the measurement results, it could be seen that the antibody of the present invention blocks the binding of HLA-G and CD8a at a level similar to or higher than that of conventional HLA-G (Table 4). These results suggest that anti-HLA-G antibodies may activate immune cells by inhibiting the binding of HLA-G and CD8a.

### Example 5: Confirmation of cancer cell death using PBMCs

SK-OV-3 cells or MDA-MB-231 cells overexpressing HLA-G (target cells) were cultured in an incubator at 37 °C for 24 hours. After the culture, the HLA-G overexpressing cells (target cells) were pretreated with the anti-HLA-G antibody at the concentration of 100 nM at 37 °C for one hour. The PBMC (effector cells) and antibody-treated target cells were mixed at a ratio of 5:1 and co-cultured with SK-OV-3 cells or MDA-MB-231 cells for 48 hours.

After the culture was completed, the cell culture solution was recovered. The level of lactate dehydrogenase (LDH) from the recovered cell culture solution was measured using a cytotoxicity assay kit (cytotox96 nonradioactive cytotoxicity assay, Promega) according to the manufacturer's protocol. Since LDH is released upon cell death, the degree of cell death may be determined by measuring the LDH level.

According to the measurement results, it could be confirmed that the anti-HLA-G antibody 2, antibody 1, and antibody 3 enhanced the cancer cell killing ability of PBMCs compared to the negative control group (IgG1 pretreatment) that does not specifically bind to HLA-G (FIG. 3).

### Example 6: Confirmation of tumor growth inhibitory effect of HLA-G antibodies

4T-1 cells overexpressing human HLA-G were transplanted into the right abdominal cavity of BALB/c mice. Tumor growth was observed 7 to 14 days after the transplantation, and the mice were raised until the tumor size grew to a certain level (100 mm³) or more. When the tumor size reached the desired size, the tumor-transplanted mice were randomly grouped according to the test groups in Table 5.

**[Table 5]**

| Test group | Administered substance | Dose (mpk) |
|---|---|---|
| Group 1 | IgG1 | 10 |
| Group 2 | Anti-HLA-G antibody 4 | 1 |
| Group 3 | Anti-HLA-G antibody 4 | 10 |
| Group 4 | Anti-HLA-G antibody 1 | 1 |
| Group 5 | Anti-HLA-G antibody 1 | 10 |
| Group 6 | Anti-HLA-G antibody 2 | 1 |
| Group 7 | Anti-HLA-G antibody 2 | 10 |
| Group 8 | Anti-HLA-G antibody 3 | 1 |
| Group 9 | Anti-HLA-G antibody 3 | 10 |

The anti-HLA-G antibody was intraperitoneally administered to each test group at a dose of 1 mg/kg (mg/kg=mpk) or 10 mg/kg, twice weekly (BIW), for a total of four times over two weeks. On day 12 from the beginning of the experiment, the volume of tumors visible under the mouse skin was measured, and the tumor growth inhibition rate was calculated according to Equation 1 below. Tumor Growth Inhibition (TGI) rate (%) = (Tumor volume of the negative control group (IgG) - Tumor volume of the test group) / Tumor volume of the negative control group

The results of the tumor growth inhibition rate calculation are shown in Table 6 below.

**[Table 6]**

| Group | Anti-HLA-G antibody | TGI (%) (BIW dose) |
|---|---|---|
| Group 4 | Antibody 1 | 20.1 (1 mpk) |
| Group 5 | Antibody 1 | 28.5 (10 mpk) |
| Group 6 | Antibody 2 | 34.4 (1 mpk) |
| Group 7 | Antibody 2 | 23.0 (10 mpk) |
| Group 8 | Antibody 3 | 22.4 (1 mpk) |
| Group 9 | Antibody 3 | 39.7 (10 mpk) |
| Group 3 | Antibody 4 | 24.9 (10 mpk) |

The negative control group, which was the IgG1 administration group, did not suppress tumor growth, whereas all anti-HLA-G antibody-treated groups suppressed tumor growth. Antibody 4 exhibited a tumor growth inhibition effect of 24.9% when administered at a dose of 10 mpk, and Antibodies 1, 2, and 3 exhibited tumor growth inhibition effects of more than 20% even when administered at a dose of 1 mpk (Table 6).

In addition, tumor tissues were isolated from the mice treated with an anti-HLA-G antibody, and immune cells that had infiltrated the tumors were analyzed. The action of immunotherapy changes the pool of immune cells in tumor tissues, and cancer cells are killed due to the activation of immune cells. Cytotoxic T cells are representative immune cells with cancer cell killing functions, and regulatory T (Treg) cells are a representative immunosuppression factor.

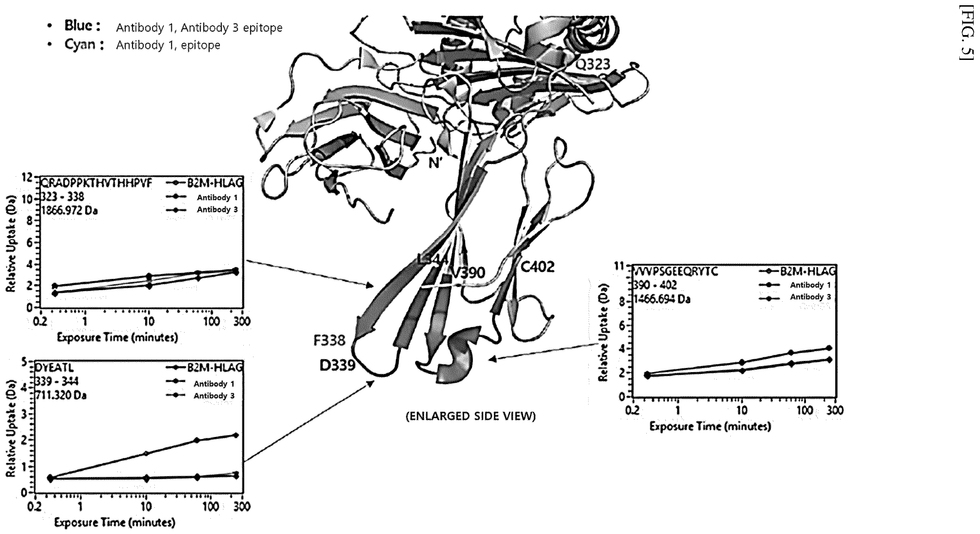

As a result of confirming the levels of cytotoxic T cells/regulatory T cells in the tumor tissues, it could be seen that the levels of cytotoxic T cells/regulatory T cells were higher in all the experimental groups administered an anti-HLA-G antibody compared to the negative control group (mock) (FIG. 5). These results mean that the anti-HLA-G antibodies increase the function of cytotoxic T cells.

### Example 7: Confirmation of binding sites of antibodies and HLA-G

Through a preliminary experiment, it was confirmed that the highest overall sequence coverage of 95.5% was achieved when a quenching buffer was used for the antigen and a pepsin solution (1:1 w/w) was treated at a low temperature for five minutes. Accordingly, an epitope mapping experiment was performed under these conditions. The quenching buffer contains 100 mM potassium phosphate, 1 M urea, and 500 mM tris(2-carboxyethyl)phosphine hydrochloride (TCEP) (pH 2.5).

The epitope mapping was performed using hydrogen deuterium exchange mass spectrometry (HDX-MS) technology, and β2M-HLA-G was used as an antigen and anti-HLA-G Antibody 1 or 2 was used as an antibody.

Samples in the state of an antigen alone and in the state of an antigen-antibody complex were each labeled with a D₂O buffer (triplicate samples were used). Thereafter, mass spectrometry was performed at five time points: 0 min, 0.33 min (20 sec), 10 min, 60 min, and 240 min, and the molecular weight values obtained through the spectra were compared to identify the portion where deuterium uptake of the complex decreased compared to the antigen alone.

As a result, it could be confirmed that the sequences of amino acids 339 to 344 (DYEATL) and amino acids 390 to 402 (VVVPSGEEQRYTC) of the antigen served as the same epitope when the antigen was bound to each of the two types of antibody. In addition, for Antibody 1, it could be seen that amino acid 323 to 338 (QRADPPKTHVTHHPVF) portion was also an epitope adjacent to amino acids 339 to 344 (FIG. 5).

## Claims

1. An anti-human leukocyte antigen-G (HLA-G) antibody or an antigen-binding fragment thereof comprising:
(a) a heavy chain complementarity determining region 1 (CDR1) including an amino acid sequence of SEQ ID NO: 1, a heavy chain CDR2 including an amino acid sequence of SEQ ID NO: 2, and a heavy chain CDR3 including an amino acid sequence of SEQ ID NO: 3; and
a light chain CDR1 including an amino acid sequence of SEQ ID NO: 5, a light chain CDR2 including an amino acid sequence of SEQ ID NO: 6, and a light chain CDR3 including an amino acid sequence of SEQ ID NO: 7;
(b) a heavy chain CDR1 including an amino acid sequence of SEQ ID NO: 9, a heavy chain CDR2 including an amino acid sequence of SEQ ID NO: 10, and a heavy chain CDR3 including an amino acid sequence of SEQ ID NO: 11; and
a light chain CDR1 including an amino acid sequence of SEQ ID NO: 13, a light chain CDR2 including an amino acid sequence of SEQ ID NO: 14, and a light chain CDR3 including an amino acid sequence of SEQ ID NO: 15;
(c) a heavy chain CDR1 including an amino acid sequence of SEQ ID NO: 17, a heavy chain CDR2 including an amino acid sequence of SEQ ID NO: 18, and a heavy chain CDR3 including an amino acid sequence of SEQ ID NO: 19; and
a light chain CDR1 including an amino acid sequence of SEQ ID NO: 21, a light chain CDR2 including an amino acid sequence of SEQ ID NO: 22, and a light chain CDR3 including an amino acid sequence of SEQ ID NO: 23; or
(d) a heavy chain CDR1 including an amino acid sequence of SEQ ID NO: 25, a heavy chain CDR2 including an amino acid sequence of SEQ ID NO: 26, and a heavy chain CDR3 including an amino acid sequence of SEQ ID NO: 27; and
a light chain CDR1 including an amino acid sequence of SEQ ID NO: 29, a light chain CDR2 including an amino acid sequence of SEQ ID NO: 30, and a light chain CDR3 including an amino acid sequence of SEQ ID NO: 31.

2. The anti-HLA-G antibody or antigen-binding fragment thereof of claim 1, wherein the HLA-G-specific antibody or antigen-binding fragment thereof comprises:
(a) a heavy chain variable region consisting of an amino acid sequence of SEQ ID NO: 4; and a light chain variable region consisting of an amino acid sequence of SEQ ID NO: 8;
(b) a heavy chain variable region consisting of an amino acid sequence of SEQ ID NO: 12; and a light chain variable region consisting of an amino acid sequence of SEQ ID NO: 16;
(c) a heavy chain variable region consisting of an amino acid sequence of SEQ ID NO: 20; and a light chain variable region consisting of an amino acid sequence of SEQ ID NO: 24; or
(d) a heavy chain variable region consisting of an amino acid sequence of SEQ ID NO: 28; and a light chain variable region consisting of an amino acid sequence of SEQ ID NO: 32.

3. The anti-HLA-G antibody or antigen-binding fragment thereof of claim 1, wherein the anti-HLA-G antibody is a recombinant antibody, a monoclonal antibody, a polyclonal antibody, a mixture of monoclonal and/or polyclonal antibodies, a human antibody, a humanized antibody, or a chimeric antibody.

4. The anti-HLA-G antibody or antigen-binding fragment thereof of claim 1, wherein the antigen-binding fragment is selected from the group consisting of fragment antigen binding (Fab), Fab', F(ab')2, a variable fragment (Fv), a disulfide-stabilized Fv fragment (dsFv), a single chain Fv (scFv), a diabody, a triabody and a tetrabody.

5. An isolated nucleic acid comprising a nucleotide sequence encoding the anti-HLA-G antibody or antigen-binding fragment thereof of claim 1.

6. A vector comprising the isolated nucleic acid of claim 5.

7. A host cell comprising the vector of claim 6.

8. A pharmaceutical composition for use in the prevention or treatment of cancer, comprising one or more active ingredients selected from the group consisting of:
(a) the anti-HLA-G binding antibody or antigen-binding fragment thereof of claim 1;
(b) the isolated nucleic acid of claim 5;
(c) the vector of claim 6; or
(d) the host cell of claim 7; and
a pharmaceutically acceptable carrier.

9. The pharmaceutical composition for use of claim 8, wherein the cancer is selected from the group consisting of pancreatic cancer, breast cancer, ovarian cancer, glioma, cervical cancer, endometrial cancer, esophageal cancer, stomach cancer, liver cancer, lung cancer, colon cancer, nasopharyngeal cancer, oral cancer, thyroid cancer, prostate cancer, renal cancer, gallbladder cancer, bile duct cancer, blood cancer, and melanoma.

10. A method for use in preventing or treating cancer, comprising administering the anti-HLA-G binding antibody or antigen-binding fragment thereof of claim 1, or the pharmaceutical composition of claim 8 to a subject in need thereof.

11. An HLA-G epitope consisting of a sequence selected from the group consisting of SEQ ID NOs: 34 to 36.

12. An antibody binding to an HLA-G epitope consisting of a sequence selected from the group consisting of SEQ ID NOs: 34 to 36.
